# EUROPÄISCHE PATENTSCHRIFT

(11) **EP 1 000 629 B1**
(45) Veröffentlichungstag und Bekanntmachung des Hinweises auf die Patenterteilung: **15.01.2003**
(21) Anmeldenummer: 99122761.2
(22) Anmeldetag: 16.11.1999
(51) Int. Cl.: A61L 2/28

(54) **Sterilisator mit einem Messwertaufnehmer und Verfahren zur Überwachung des Messwertaufnehmers**
Steriliser with a sensor and method for monitoring the sensor
Stérilisateur équipé d'un capteur et procédé de surveillance de ce capteur

(30) Priorität: 16.11.1998 DE 19852793
(43) Veröffentlichungstag der Anmeldung: 17.05.2000
(73) Patentinhaber: MMM MÜNCHENER MEDIZIN MECHANIK GMBH, 82152 Planegg (DE)
(72) Erfinder: Hofmann, Ludwig, Dipl.-Ing., 81377 München (DE); Krotz, Martin, Dipl.-Ing., 81371 München (DE); Büchner, Robert, 85614 Kirchseeon (DE)
(74) Vertreter: Grünecker, Kinkeldey, Stockmair & Schwanhäusser Anwaltssozietät

(56) Entgegenhaltungen:
- WO-A-93/21964
- WO-A-97/47331
- WO-A-98/00176
- US-A- 5 164 161
- US-A- 5 880 438

## Beschreibung

Die Erfindung betrifft einen Sterilisator mit einem Meßwertaufnehmer sowie ein Verfahren zur Überwachung eines entsprechenden Meßwertaufnehmers.

Zur Aufbereitung von Sterilgut beispielsweise im medizinischen Bereich wird dieses üblicherweise in eine ggf. beheizte Sterilisatorkammer eines Sterilisators verbracht und in dieser Sterilisatorkammer über eine vorbestimmte Zeit einem Prozeßmedium, beispielsweise Dampf, oder einer toxischen Gasmischung ausgesetzt. Der hierbei erreichte Sterilisationserfolg hängt neben der Behandlungsdauer innerhalb der Sterilisatorkammer insbesondere von der Temperatur des Prozeßmediums und dem zeitlichen Verlauf des in der Kammer herrschenden Druckes ab. Die genannten Größen Druck und Temperatur werden über Meßwertgeber erfaßt und an eine Steuereinrichtung weitergeleitet. In Abhängigkeit von den erfaßten Meßwerten steuert die Steuereinrichtung beispielsweise den Zustrom, den Abstrom und die Temperatur des Prozeßmediums sowie die Temperatur des Mantels der Sterilisatorkammer und arbeitet dabei vollautomatisch einen vorbestimmten Behandlungsablauf ab. Bedarfsweise kann mit Blick auf einen späteren Nachweis einer hinreichenden Behandlung des Sterilgutes der Verlauf der jeweiligen Sterilisationsbehandlung aufgezeichnet werden. Hierzu werden beispielsweise der zeitliche Verlauf der Temperatur des Prozeßmediums, die Temperatur der Wandung der Sterilisatorkammer, der zeitliche Verlauf des Druckes des Prozeßmediums und ggf. die Zusammensetzung des Prozeßmediums dokumentiert. Eine entsprechende Dokumentationseinrichtung kann unmittelbar mit der Steuereinrichtung in Datenverbund stehen bzw. in diese integriert sein. Die der Dokumentation zugeführten Meßdaten können über die zur Steuerung des Sterilisators vorgesehenen Meßwertaufnehmer gewonnen werden. Da die Steuerung des Sterilisators und der Sterilisationserfolg wesentlich von der Funktionsfähigkeit und der Genauigkeit der genannten Meßwertaufnehmer abhängt, werden diese in vorbestimmten Zeitintervallen durch herstellerseitig kalibrierte und geeichte Meßwertaufnehmer ausgetauscht. Diese Meßwertaufnehmer sind daher üblicherweise gut zugänglich angeordnet, so daß eine einfache Austauschbarkeit derselben gewährleistet ist. Der Einbau eines kalibrierten Meßwertaufnehmers erfordert jedoch besondere Sorgfalt, da u.U das Meßwertaufnahmeverhalten des Meßwertaufnehmers durch den Einbauvorgang und ggf. durch die Verlegung der entsprechenden Verbindungsleitungen beeinflußt wird.

WO 9800176 offenbart einen Sterilisator, der eine Aufnahme für ein Halbleitersensorelement besitzt und ein zweites externes Sensorelement, das außerhalb der Sterilisier-Kammer angeordnet ist.

DE 3138046 offenbart ein Meßgerät sowie ein Verfahren zum Einschreiben von Korrelationsdaten in dieses Meßgerät, die zum Eichen des Meßgerätes dienen.

Das Verfahren beschreibt die Durchführung eines einmaligen Eichschrittes unter standardisierten Bedingungen während der Herstellung, bei dem ein zweiter Meßfühler angeschlossen wird.

Der Erfindung liegt die Aufgabe zugrunde, die Durchführung einer vorgegebenen Sterilisationsbehandlung in einem mit Blick auf die Abweichungen der Prozeßparameter gegenüber vorgegebenen Sollwerten engen Toleranzbereich auf zuverlässige Weise unter einem verringerten Wartungs- und Kostenaufwand zu gewährleisten.

Diese Aufgabe wird erfindungsgemäß durch einen Sterilisator mit den in Patentanspruch 1 angegebenen Merkmalen bzw. durch das in Patentanspruch 11 angegebene Verfahren gelöst.

Dadurch wird es auf vorteilhafte Weise möglich, das Meßwertaufnahmeverhalten des in dem Sterilisator in Funktionsstellung eingebauten Meßwertaufnehmers zu erfassen. Die Gefahr, daß das tatsächliche Meßwertaufnahmeverhalten des Meßwertaufnehmers von dem Meßwertaufzeichnungsverhalten unter den herstellerseitig berücksichtigten Elch-Bedingungen abweicht, ist damit weitgehend gebannt. Zudem besteht die Möglichkeit, etwaige alterungsbedingte Änderungen des Aufzeichnungsverhaltens des Meßwertaufnehmers seitens der Steuereinrichtung durch entsprechende Aktualisierung von Auswertungsprozeduren zu kompensieren. Auf einen Austausch eines an sich funktionsfähigen, jedoch lediglich hinsichtlich seines Aufzeichnungsverhaltens veränderten Meßwertaufnehmer kann auf vorteilhafte Weise verzichtet werden. Hierdurch wird eine erhebliche Reduzierung der Wartungskosten erreicht. Weiterhin wird es auf vorteilhafte Weise möglich, das Alterungsverhalten des Meßwertaufnehmers unmittelbar über die Steuereinrichtung zu erfassen, wodurch es möglich wird, die voraussichtliche Lebensdauer des Meßwertaufnehmers zu bewerten, und einen geeigneten Zeitpunkt für das Auswechseln des Meßwertaufnehmers zu bestimmen. Insgesamt wird hierdurch eine erhebliche Verringerung des Wartungsaufwandes eines Sterilisators erreicht.

Bei dem genannten Meßwertaufnehmer kann es sich um einen Drucksensor, um einen Temperatursensor, Meßwertaufnehmer für Feuchte, Konzentrationen oder Inertgaszuanteile im Dampf sowie um Sensoren zur Erfassung eines Gas-Volumenstromes oder Meßwertaufnehmer zur Beurteilung des Dampfzustandes handeln. Der Meßwertaufnehmer und der Referenzmeßwertaufnehmer können an sich bauartidentisch sein oder zumindest nach dem gleichen Meßprinzip arbeiten. Es ist jedoch auch möglich, als Referenzmeßwertaufnehmer einen Meßwertaufnehmer zu verwenden, der innerhalb eines größeren Meßbereiches einen oder vorzugsweise wenigstens zwei Referenzmeßwerte mit besonders hoher Genauigkeit liefert. Anhand derartiger punktueller und mit hoher Zuverlässigkeit ermittelter Referenzmeßwerte kann die Grundjustierung ("Nullpunkt") des zu überprüfenden Meßwertaufnehmers eingestellt werden.

Eine mit Blick auf eine besonders rasche Überprüfung und ggf. erforderliche Korrektur des ersten Meßwertaufnehmers vorteilhafte Ausführungsform der Erfindung ist dadurch gegeben, daß die Steuereinrichtung eine Datenverarbeitungseinrichtung umfaßt, welche die Meßwertauswertung der Meßwerte des ersten Meßwertaufnehmers auf Grundlage der durch den Referenzmeßwertaufnehmer aufgenommenen Meßwerte aktualisiert oder korrigiert. Eine derartige Aktualisierung bzw. Korrektur der programmgesteuert erfolgenden Meßwerterfassung und Meßwertauswertung der Meßwerte des ersten Meßwertaufnehmers kann auf vorteilhafte Weise auf Grundlage einer Korrekturfunktion, insbesondere eines, durch mehrere Stützstellen definierten Korrekturpolynoms erfolgen, das die Abweichungen zwischen den korrespondierenden Meßwerten des ersten Meßwertaufnehmers und des Referenzmeßwertaufnehmers ausgleicht.

Im Hinblick auf eine zuverlässige Erfassung etwaiger Funktionsstörungen des ersten Meßwertaufnehmers ist in vorteilhafter Weise eine Prüfeinrichtung vorgesehen, die eine Abweichung zwischen den durch den ersten Meßwertaufnehmer aufgenommenen Meßwerten und den durch den Referenzmeßwertaufnehmer aufgenommenen Referenzmeßwerten mit vorgegebenen Referenz-Abweichungen vergleicht. Die Referenz-Abweichungen können aus einer entsprechenden Referenz-Funktion errechnet oder als entsprechender Datensatz in der Steuereinrichtung bzw. in der Datenverarbeitungseinrichtung gespeichert sein, und können insbesondere für unterschiedliche Meßwerte unterschiedlich groß sein. Beispielsweise können für die Randpunkte des Meßbereiches höhere Genauigkeiten gefordert werden als für den dazwischenliegenden Bereich. Die Prüfeinrichtung zur Überprüfung der Größe der Abweichungen zwischen den durch den Meßwertaufnehmer aufgenommenen Meßwerten und den durch den Referenzmeßwertaufnehmer aufgenommenen Referenzmeßwerten ist in vorteilhafter Weise unmittelbar in die Datenverarbeitungseinrichtung integriert. Es ist auch möglich, die Prüfeinrichtung und die Datenverarbeitungseinrichtung separat von der zur Steuerung des Sterilisators vorgesehenen Steuereinrichtung auszubilden und nur im Rahmen der Überprüfung des ersten Meßwertaufnehmers mit der Steuereinrichtung des Sterilisators zu verbinden. Die Durchführung der Überprüfung des ersten, eingebauten Meßwertaufnehmers im Rahmen eines Prüfvorganges wird vorzugsweise dokumentiert, indem sowohl die durch den ersten Meßwertaufnehmer aufgenommenen Meßwerte als auch die hierzu korrespondierend durch den Referenzmeßwertaufnehmer aufgenommenen Meßwerte protokolliert werden. Die jeweils aufgenommenen Meßwerte werden vorzugsweise auf einer Datenausgabeeinrichtung, beispielsweise in Form eines Druckers oder eines LCD-Displays, graphisch dargestellt. Auch die auf Grundlage der ermittelten Meßwerte errechnete Korrekturfunktion wird in vorteilhafter Weise graphisch dargestellt.

Die Steuereinrichtung des Sterilisators, die Datenverarbeitungseinrichtung oder die Prüfeinrichtung sind vorzugsweise derart ausgebildet, daß nach Korrektur bzw. Abgleich der zur Meßwertauswertung der Meßwerte des ersten Meßwertaufnehmers vorgesehenen Auswertungsprozeduren ein Prüfablauf veranlaßt wird, durch welchen die durch die aktualisierte Meßwertauswertung der Meßwerte des ersten Meßwertaufnehmers ermittelten Daten mit den durch den Referenzmeßwertaufnehmer aufgenommenen korrespondierenden Meßwerten verglichen werden.

Der Sterilisator ist in vorteilhafter Weise derart ausgebildet, daß der Referenzmeßwertaufnehmer auf einfache Weise an dem Sterilisator anbringbar ist und nach Abschluß einer entsprechenden Sterilisatorprüf-Prozedur einfach aus dem Sterilisator entfernt werden kann. Die Steuereinrichtung des Sterilisators ist vorzugsweise mit einer entsprechenden Schnittstelle, beispielsweise einer seriellen Schnittstelle, versehen, über welche der Referenzmeßwertaufnehmer bzw. ein Vorschaltgerät desselben mit der Steuereinrichtung koppelbar ist.

Es ist jedoch auch möglich, eine Datenverbindung bzw. Datenauswertung der durch den ersten Meßwertaufnehmer und den Referenzmeßwertaufnehmer aufgenommenen Meßwerte über ein Netzwerk vorzunehmen. Hierzu ist es beispielsweise möglich, den Referenzmeßwertaufnehmer mit einem vorzugsweise tragbaren PC zu verbinden und diesen PC wiederum entweder über das genannte Netzwerk oder über eine entsprechende Datenleitung mit der Steuereinrichtung des Sterilisators zu verbinden. Dieser PC kann für den entsprechenden Sterilisatorprüf-Vorgang ggf. die wesentlichen Funktionen der Steuereinrichtung ebenfalls übernehmen.

In dem genannten PC kann ein entsprechendes Sterilisator-Prüfprogramm gespeichert sein, das eine mit Blick auf eine besonders günstige Ermittlung der Referenzmeßwerte geeignete Ansteuerung des Sterilisators veranlaßt. Durch den PC kann weiterhin ein Prüfprogramm abgearbeitet werden, das die im Rahmen der Sterilisatorprüfung durch die beiden Meßwertaufnehmer ermittelten Meßdaten vergleicht und ggf. entsprechende Korrekturdaten errechnet. Die durch den PC ermittelten Korrekturdaten können an die Steuereinrichtung übertragen werden.

Durch die derart vorgenommene Hardware-Trennung, der zur Änderung der Meßwertauswertung der Sterilisator-Steuereinheit erforderlichen Komponenten von der Sterilisator-Steuereinheit wird auf vorteilhafte Weise eine unbefugte Änderung der Sterilisator-Steuereinrichtung verhindert.

Es ist jedoch auch möglich, sämtliche zur Aktualisierung der sterilisatorseitig vorgenommenen Meßwertauswertung erforderlichen Funktionen durch die Sterilisator-Steuereinrichtung abzuwickeln. Eine unbefugte Änderung der zur Meßwertauswertung erforderlichen Umrechnungsdaten wird hierbei vorzugsweise durch Abfrage einer Zugangsberechtigung verhindert. Ein entsprechender Sterilisator-Kontrollvorgang kann vorzugsweise ebenfalls nur nach erfolgreicher Überprüfung einer Zugangsberechtigung vorgenommen werden.

Das erfindungsgemäße Verfahren zur Überwachung des Meßwertaufnehmers eines Sterilisators wird vorzugsweise mehrfach durchgeführt. Die Anzahl der erforderlichen Prüfzyklen wird in vorteilhafter Weise in Abhängigkeit von den Abweichungen zwischen den durch den ersten Meßwertaufnehmer und den Referenzmeßwertaufnehmer ermittelten Meßwerten bestimmt.

In vorteilhafter Weise ist es möglich, bei der Durchführung mehrerer Kontrollzyklen bestimmte weitere Parameter zu verändern. Beispielsweise ist es möglich, die Überprüfung eines zur Temperaturerfassung vorgesehenen Meßwertaufnehmers bei unterschiedlichen Dampfdurchflußgeschwindigkeiten und unterschiedlichen Systemdrücken vorzunehmen. Auf Grundlage der durch den Referenzmeßwertaufnehmer erfaßten Meßwerte kann dann das Meßwertaufnahmeverhalten des ersten Meßwertaufnehmers in Abhängigkeit mehrerer Systemparameter bestimmt werden. Dadurch wird es möglich, ein Korrekturkennfeld zu ermitteln, auf dessen Grundlage eine besonders präzise Meßwertauswertung vorgenommen werden kann.

Weitere vorteilhafte Weiterbildungen der Erfindung sind in den Unteransprüchen angegeben.

Weitere Einzelheiten und Merkmale der Erfindung ergeben sich aus der nachfolgenden Beschreibung unter Bezugnahme auf die beigefügten Zeichnungen. Es zeigen:
- Fig. 1: eine vereinfachte Schemadarstellung eines Sterilisators nebst zugehörigen Steuerungs- und Versorgungseinrichtungen;
- Fig. 2: ein Flußdiagramm zur Erläuterung des erfindungsgemäßen Verfahrens zur Überwachung eines Meßwertaufnehmers.
- Fig. 3: eine vereinfachte Axialschnittansicht einer Meßwertaufnehmeranordnung; und

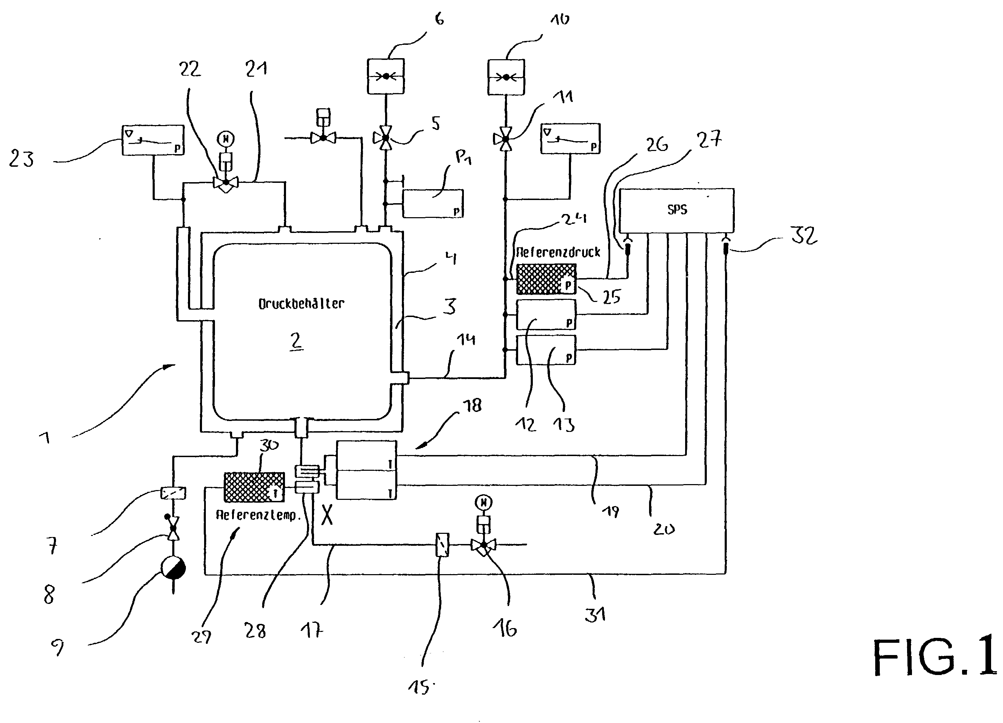

Die Darstellung gemäß Fig. 1 zeigt einen Sterilisator 1 mit einer Sterilisatorkammer 2. Die Sterilisatorkammer 2 ist unter Bildung eines Mantelraumes 3 von einem Kammermantel 4 umgeben.

Der Mantelraum 3 ist über eine Heizventileinrichtung 5 mit einer Dampfquelle 6 verbindbar. Der Öffnungsquerschnitt einer in der Heizventileinrichtung 5 gebildeten Durchgangsöffnung ist nach Maßgabe einer Steuereinheit SPS einstellbar. Durch den entsprechend gesteuert aus der Dampfquelle 6 entnommenen Dampf ist der dem Mantelraum 3 zugeführte Dampfstrom steuerbar. Der in dem Mantelraum 3 herrschende Druck wird über einen Drucksensor P1 erfaßt. Der Drucksensor P1 steht über eine hier nicht gezeigte Verbindungsleitung mit der Steuereinheit SPS in Verbindung. Über einen Dampffilter 7, eine nachgeschaltete Ablaßventileinrichtung 8 und einen Kondensatabscheider 9 kann der in dem Mantelraum 3 befindliche Heizdampf sowie etwaiges sich darin bildendes Kondensat abgeleitet werden.

Zur Steuerung der Zufuhr eines aus einer Sterilisierdampf-quelle 10 entnommenen Sterilisierdampfes ist eine Sterildampfventileinrichtung 11 vorgesehen, deren Stellglied ebenfalls über eine hier nicht gezeigte Verbindungsleitung mit der Steuereinheit SPS verbunden ist, so daß ein in der Sterildampfventileinrichtung 11 begrenzter Ventildurchgangsquerschnitt nach Maßgabe eines durch die Steuereinheit SPS erzeugten Stellwertes veränderbar ist.

Zur Erfassung des Dampfdruckes in einem in Dampfstromrichtung der Sterildampfventileinrichtung abfolgenden Rohrleitungsabschnitt 14 sind zwei, hier als Drucksensoren 12 und 13 ausgebildete Meßwertaufnehmer vorgesehen. Die durch diese Drucksensoren 12, 13 erzeugten Meßsignale werden der Steuereinheit SPS zugeführt. Durch die Verwendung von zwei Drucksensoren zur Erfassung des in dem genannten Rohrleitungsabschnitt herrschenden Druckes kann der entsprechende Dampfdruck auch bei Ausfall eines der beiden Sensoren erfaßt und das System in einen stabilen Zustand überführt werden.

Der über die Sterildampfventileinrichtung 11 dosiert der Sterilisierdampfquelle 10 entnommene Sterilisierdampf gelangt über den genannten Rohrleitungsabschnitt 14 in die Sterilisatorkammer 2 und kann hierbei mit dem entsprechend in der Sterilisatorkammer 2 aufgenommenen Sterilgut in Kontakt treten. Über eine Dampffiltereinrichtung 15 und über eine nachgeschaltete Ablaßventileinrichtung 16 kann der in der Sterilisatorkammer 2 befindliche Dampf abgeleitet werden. In einem zwischen der Sterilisatorkammer 2 und der Ablaßventileinrichtung 16 ausgebildeten Ablaßrohrleitungsabschnitt 17 ist ein Meßwertaufnehmer 18 vorgesehen zur Erfasung der Temperatur des über den Ablaßrohrleitungsabschnitt abgeleiteten Dampfes. Der Meßwertaufnehmer 18 umfaßt zwei Temperatursensoren, die über entsprechende Verbindungsleitungen 19 und 20 mit der Steuereinheit SPS verbunden sind.

Die Sterilisatorkammer 2 ist über eine Überbrückungsleitung 21 mit dem Mantelraum 3 des Sterilisators verbindbar. In dieser Überbrückungsleitung 21 ist ein Überbrückungsventil 22 vorgesehen, das ebenfalls über eine nicht gezeigte Verbindungsleitung nach Maßgabe der Steuereinheit SPS ansteuerbar ist. Durch entsprechende Ansteuerung des Überbrückungsventiles 22 und Druckbeaufschlagung der Sterilisatorkammer 2 und des Mantelraumes 3 ist es möglich, Dampf aus dem Mantelraum 3 in die Sterilisatorkammer 2 einzuleiten. Prinzipiell ist es auch möglich, Dampf aus der Sterilisatorkammer 2 in den Mantelraum 3 einzuleiten oder durch entsprechendes Öffnen des Überbrückungsventiles 22 Druckgleichheit zwischen Mantelraum 3 und Sterilisatorkammer 2 zu erzeugen. Dadurch wird es möglich, mit einem einzigen Referenzmeßwertaufnehmer mehrere für den Regelbetrieb vorgesehene Meßwertaufnehmer, beispielsweise mehrere Drucksensoren, gleichzeitig zu überprüfen.

Der Absolutdruck im Inneren der Sterilisisatorkammer 2 ist über einen Meßwertaufnehmer 23, der hier einen Drucksensor umfaßt, erfaßbar. Der Meßwertaufnehmer 23 steht ebenfalls über eine hier nicht gezeigte Verbindungsleitung mit der Steuereinheit SPS in Verbindung. In dem zur Zufuhr des Sterilisierdampfes zu der Sterilisatorkammer 2 vorgesehenen Rohrleitungsabschnitt 14 ist ein Anschlußstutzen 24 angeordnet, an welchen ein hier durch Rautenschraffur gekennzeichneter Referenzmeßwertaufnehmer 25 anschließbar ist. Bei dem zum Anschluß an den Anschlußstutzen 24 vorgesehenen Referenzmeßwertaufnehmer 25 handelt es sich im vorliegenden Falle um einen hochpräzise geeichten Drucksensor, der über eine Prüfverbindungsleitung 26 und eine Prüf-Schnittstelleneinrichtung 27 (serielle Schnittstelle) mit der Steuereinheit SPS verbindbar ist.

In dem zwischen der Sterilisatorkammer 2 und der Ablaßventileinrichtung 16 angeordneten Ablaßrohrleitungsabschnitt 17 ist ebenfalls ein Anschlußstutzen 28 vorgesehen zur Bildung einer Referenzmeßstelle, an welche ein Referenzmeßwertaufnehmer 29 anschließbar ist.

Bei dem hier an den Anschlußstutzen 28 anschließbaren Referenzmeßwertaufnehmer 29 handelt es sich um einen hochpräzise geeichten Referenztemperatursensor 30. Dieser Referenztemperatursensor 30 ist über eine Verbindungsleitung 31 sowie eine Prüfschnittstelleneinrichtung 32 mit der Steuereinheit SPS verbindbar.

Im Normalbetrieb des Sterilisators sind die beiden Referenzmeßwertaufnehmer 25 und 29 demontiert und die entsprechenden Anschlußstutzen 24 und 28 durch Blindstopfen verschlossen.

Während des Sterilisator-Regelbetriebes werden die zur Systemsteuerung erforderlichen Druckwerte über die in dem Rohrleitungsabschnitt 14 parallel angeordneten Drucksensoren 12 und 13, den in der Überbrückungsleitung 21 angeordneten Meßwertaufnehmer 23 sowie den in der Mantelraumversorgungsleitung angeordneten Drucksensor P1 ermittelt. Die Temperatur des in der Sterilisatorkammer 2 befindlichen Dampfes wird im Regelbetrieb über den in dem Rohrleitungsabschnitt 17 angeordneten Meßwertaufnehmer 18 erfaßt. Der Meßwertaufnehmer 18 umfaßt zwei parallel angeordnete Temperatursensoren gleicher Bauart zur Erfassung der Dampftemperatur.

Zur Überprüfung der Meßwertaufnehmer 18, 23 sowie 12 und 13 werden die an den Anschlußstutzen 24 und 28 vorgesehenen Blindstopfen entfernt und in den Anschlußstutzen 24 wird der Referenzmeßwertaufnehmer 25 eingeschraubt. In den Anschlußstutzen 28 wird der Referenzmeßwertaufnehmer 29 eingeschraubt. Beide Referenzmeßwertaufnehmer 25 und 29 werden über die genannten Prüfschnittstelleneinrichtungen 27 und 32 unmittelbar mit der Steuereinheit SPS verbunden.

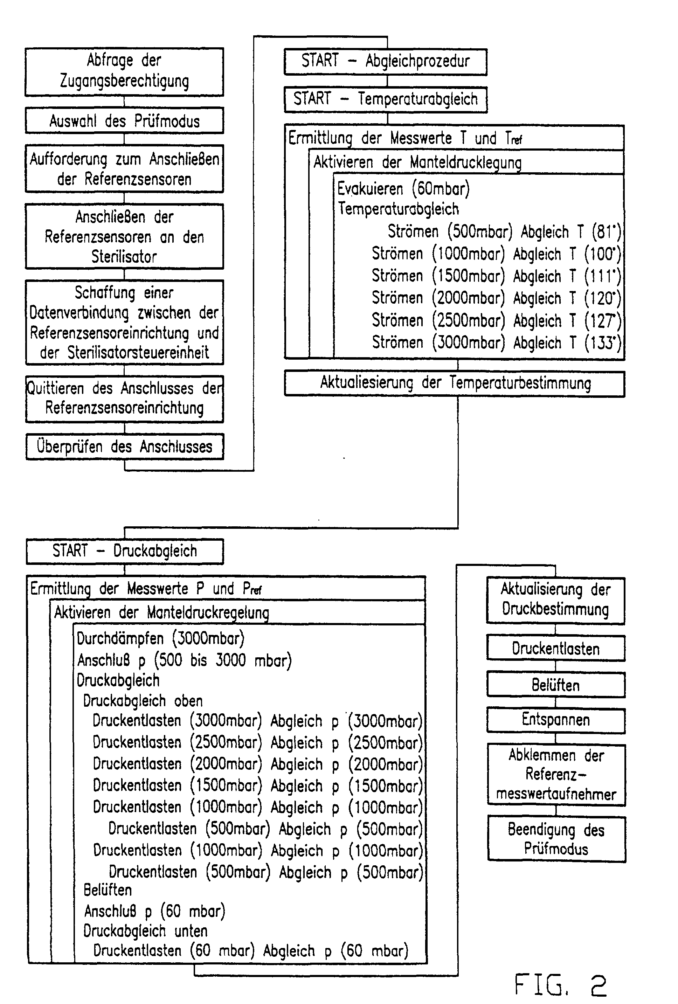

Der mit einer derartigen Systemkonfiguration durchführbare System-Prüfzyklus wird nachfolgend unter Bezugnahme sowohl auf Fig. 1 als auch auf Fig. 2 beschrieben.

Nach Anschluß der Referenzmeßwertaufnehmer 25 und 29 an die Rohrleitungsabschnitte 14 und 17 und Anschließen der Verbindungsleitungen 26 und 31 an die Steuereinheit SPS wird über eine ebenfalls an die Steuereinheit SPS angeschlossene (hier nicht dargestellte) Eingabeeinrichtung in die Steuereinheit SPS ein Paßwort eingegeben und die Steuereinheit SPS zur Abarbeitung einer Überwachungsprozedur veranlaßt.

Im Rahmen dieser Überwachungsprozedur wird zunächst das Temperatur-Meßwertaufnahmeverhalten der Meßwertaufnehmer 18 ermittelt. Hierzu werden die Ventileinrichtungen 5 und 11 und die Ventileinrichtung 22 entsprechend angesteuert. Der über die Dampfquellen 6 und 10 dem Sterilisator zuströmende Dampf gelangt über die Sterilisatorkammer 2 in den Ablaßrohrleitungsabschnitt 17 und entströmt dieser ggf. über die Ablaßventileinrichtung 16. Der durch den Ablaßrohrleitungsabschnitt 17 strömende Dampf überströmt die Temperatursensoren des Meßwertaufnehmers 18. Die von den beiden Temperatursensoren des Meßwertaufnehmers 18 aufgenommenen Meßwerte werden separat über die Verbindungsleitungen 19, 20 der Steuereinheit SPS zugeführt. Der über die Temperatursensoren des Meßwertaufnehmers 18 strömende Dampf überströmt ferner den in den Anschlußstutzen 28 hineinragenden Referenztemperatursensor 30 des Referenzmeßwertaufnehmers 29. Der durch den Referenzmeßwertaufnehmer 29 ermittelte Meßwert wird ebenfalls an die Steuereinheit SPS weitergeleitet. Bei Erreichen eines bestimmten, von dem Referenzmeßwertaufnehmer 29 erfaßten Temperaturwertes werden die beiden durch den Meßwertaufnehmer 18 erfaßten Temperaturmeßwerte gemeinsam mit dem Referenztemperaturmeßwert sofort verglichen und ggf. gespeichert. Im Falle einer unzulässig hohen Abweichung wird der entsprechende Temperatur-Meßvorgang nochmals wiederholt.

Anschließend werden die Ventile 5 und 11 derart angesteuert, daß ein weiterer Temperaturanstieg des über den Ablaßrohrleitungsabschnitt 17 abströmenden Dampf erreicht wird. Bei Erreichen eines weiteren, durch den Referenzmeßwertaufnehmer 29 erfaßten höheren Temperaturmeßwert werden wiederum die beiden durch den Meßwertaufnehmer 18 ermittelten Temperaturwerte verglichen und ggf. gemeinsam mit dem entsprechenden Referenztemperaturwert gespeichert.

Durch sukzessive Steigerung der Temperatur des aus dem Ablaß-Rohrleitungsabschnitt 17 abgeleiteten Dampf werden in Schritten von beispielsweise 5K zu den sukzessive erreichten Referenztemperaturen die zugehörigen, durch den Meßwertaufnehmer 18 ermittelten Temperaturmeßwerte aufgezeichnet. Dieser Vorgang wird solange fortgesetzt, bis die maximale Betriebstemperatur erreicht ist.

Anschließend werden die durch den Meßwertaufnehmer 18 ermittelten Temperaturmeßwerte mit den zugehörigen Referenztemperaturen verglichen. Falls die ermittelten Abweichungen einen zugelassenen Höchstwert überschreiten, wird dieser Umstand über eine Anzeigeeinrichtung, beispielsweise einem LCD-Bildschirm, einem entsprechenden Servicetechniker angezeigt.

Sofern sich die Abweichungen innerhalb eines zulässigen Toleranzbereiches bewegen, wird eine Korrekturfunktion errechnet, durch welche im späteren Regelbetrieb des Sterilisators die durch den Meßwertaufnehmer 18 gewonnenen Temperaturmeßwerte korrigiert werden.

Diese Korrekturfunktion wird in der Steuereinheit SPS gespeichert.

Der vorangehend beschriebene Überprüfungszyklus zur Überprüfung des Meßwertaufnahmeverhaltens der Meßwertaufnehmer 18 kann beispielsweise in Abhängigkeit von der Größe der ermittelten Abweichungen mehrfach durchgeführt werden. Auf Grundlage der im Rahmen mehrerer Prüfzyklen ermittelter korrelierender Meßwerte kann die entsprechende Korrekturfunktion noch weiter optimiert werden.

Im Anschluß an den Prüfzyklus zur Überprüfung des Meßwertaufnahmeverhaltens der Meßwertaufnehmer 18 können sämtliche Drucksensoren des Sterilisators anhand des in den Anschlußstutzen 24 eingeschraubten Referenzmeßwertaufnehmer 25, der hier einen Drucksensor umfaßt, überprüft werden.

Hierzu werden zunächst sämtliche Ventileinrichtungen 5, 11, 16 und 22 in einen Schließzustand gebracht. Anschließend wird die Ventileinrichtung 16 geöffnet und über eine der Ventileinrichtung 16 abfolgend angeordnete Vakuumpumpe (nicht dargestellt) der Druck im Inneren der Sterilisatorkammer 2 auf einen über den Meßwertaufnehmer 25 erfaßten Referenzdruck von 60 mbar abgesenkt. Sowohl der Meßwertaufnehmer 23 als auch die Meßwertaufnehmer 12 und 13 stehen mit der Sterilisatorkammer 2 in Verbindung. Die durch die Meßwertaufnehmer 23, 12 und 13 erfaßten Druckwerte werden in der Steuereinheit SPS unter Zuordnung zu dem gleichzeitig seitens des Referenzmeßwertaufnehmers 25 ermittelten Druckwertes gespeichert.

Anschließend wird die Sterilisierdampfventileinrichtung nach Maßgabe der Steuereinheit SPS in eine Offenstellung gebracht. Über die Sterilisierdampfquelle 10 gelangt kontinuierlich Dampf in die Sterilisatorkammer 2. Der Druck im Inneren der Sterilisatorkammer 2 steigt hierbei an. Sobald der Druck im Inneren der Sterilisatorkammer 2 einen seitens des Referenzmeßwertaufnehmers 25 erfaßten, um einen vorbestimmten Druckpegel (z.B. 500 mbar) erhöhten Druckwert erfaßt, werden erneut die zeitgleich von den Meßwertaufnehmern 23, 12 und 13 erfaßten Druckwerte mit dem zugehörigen Referenzdruck abgespeichert. Die Aufzeichnung der sukzessive zu einem bestimmten Referenzdruckpegel erfaßten Druckwerte der Meßaufnehmer 23, 12 und 13 wird solange fortgesetzt, bis der Druck im Inneren der Sterilisatorkammer 2 im wesentlichen dem Umgebungsdruck entspricht. Anschließend wird das Überbrückungsventil 22 geöffnet, wodurch ein Druckausgleich zwischen dem Mantelraum 3 und der Sterilisatorkammer 2 erreicht wird. Durch das Öffnen des Überbrückungsventils 22 wird der Drucksensor P1 ebenfalls in den Sterilisatorprüfzyklus mit einbezogen. Anschließend wird die Ablaßventileinrichtung 16 geschlossen. Durch kontinuierliches Nachströmen des Sterilisierdampfes über den Rohrleitungsabschnitt 14 wird der Druck im Inneren der Sterilisatorkammer 2 weiter erhöht. Der über den Rohrleitungsabschnitt 14 zuströmende Dampfstrom ist derart gering eingestellt, daß im gesamten Druck-System weitgehend Druckgleichheit herrscht.

Im Rahmen der weiteren Erhöhung des Druckes im Inneren der Sterilisatorkammer werden weiterhin eine Vielzahl Meßwerte bestimmt, die dem jeweiligen Referenzmeßwert zugeordnet werden. Bei Erreichen eines weiteren, bestimmten Überdruckpegels veranlaßt die Steuereinheit SPS Einschließen des Überbrückungsventils 22. Der Drucksensor P1 wird von der nachfolgenden Überprüfung ausgeschlossen.

Der Druck im Inneren der Sterilisatorkammer 2 wird sukzessive auf den maximal zulässigen Druck erhöht. Hierbei werden wiederum die entsprechenden, über die Drucksensoren 23, 12 und 13 erfaßten Druckpegel unter Zuordnung zu dem jeweiligen Referenzdruckpegel, gespeichert. Nach Erreichen des maximal zulässigen Druckes veranlaßt die Steuereinheit SPS ein Schließen der Sterildampfventileinrichtung 11 und ein Öffnen der Ablaßventileinrichtung 16. Die im Rahmen der nunmehr folgenden Druckabsenkung seitens des Referenzdrucksensors und der Drucksensoren 23, 12 und 13 erfaßten Meßwerte werden wiederum durch die Steuereinheit SPS gespeichert.

Vermittels einer der Steuereinheit SPS zugeordneten Datenverarbeitungseinrichtung werden die erfaßten Druckwerte mit den zugehörigen Referenzdruckwerten verglichen. Im Falle einer unzulässig hohen Abweichung zwischen den zugehörigen Druckwerten wird unter Angabe des entsprechenden Drucksensors ein Warnsignal ausgegeben.

Falls die ermittelten Abweichungen der erfaßten Druckpegel gegenüber dem zugehörigen Referenzdruckpegel sich innerhalb eines zugelassenen Tolerenzbereiches befinden, wird für jeden Drucksensor eine Korrekturfunktion errechnet, die anschließend in der Speichereinrichtung abgespeichert wird. Falls die ermittelten Abweichungen sich innerhalb eines vorbestimmten, sehr engen Tolerenzbereiches befinden, wird seitens der Steuereinheit signalisiert, daß der Prüfzyklus erfolgreich abgeschlossen wurde. Falls die ermittelten Abweichungen außerhalb dieses engen Toleranzbereiches jedoch innerhalb des zulässigen Toleranzbereiches liegen, veranlaßt die Steuereinheit SPS die Durchführung eines nochmaligen Prüf-Zyklus.

Sobald feststeht, daß auf Grundlage der zu jedem Meßwertaufnehmer aktualisierten Meßwertauswertungsfunktion eine hinreichend genaue Steuerung des Sterilisators möglich ist, wird der Sterilisator-Prüfzyklus beendet.

Im Rahmen der Beendigung des Sterilsatorprüf-Zyklus können über einen an die Steuereinheit SPS angeschlossenen Drucker die erfaßten Daten und die darauf basierend errechneten Korrekturfunktionen ausgegeben, insbesondere graphisch dargestellt werden. Nach Abschluß des Sterilisatorprüf-Zyklus werden die Referenzmeßwertaufnehmer 25 und 29 aus den entsprechenden Anschlußstutzen 24 und 28 entfernt und von der Steuereinheit SPS abgezogen.

Durch das Abziehen der Verbindungsleitungen der Referenzmeßwertaufnehmer von der Sterilisatorsteuereinheit SPS wird automatisch die Steuereinheit SPS in den Regelbetriebsmodus zurückversetzt.

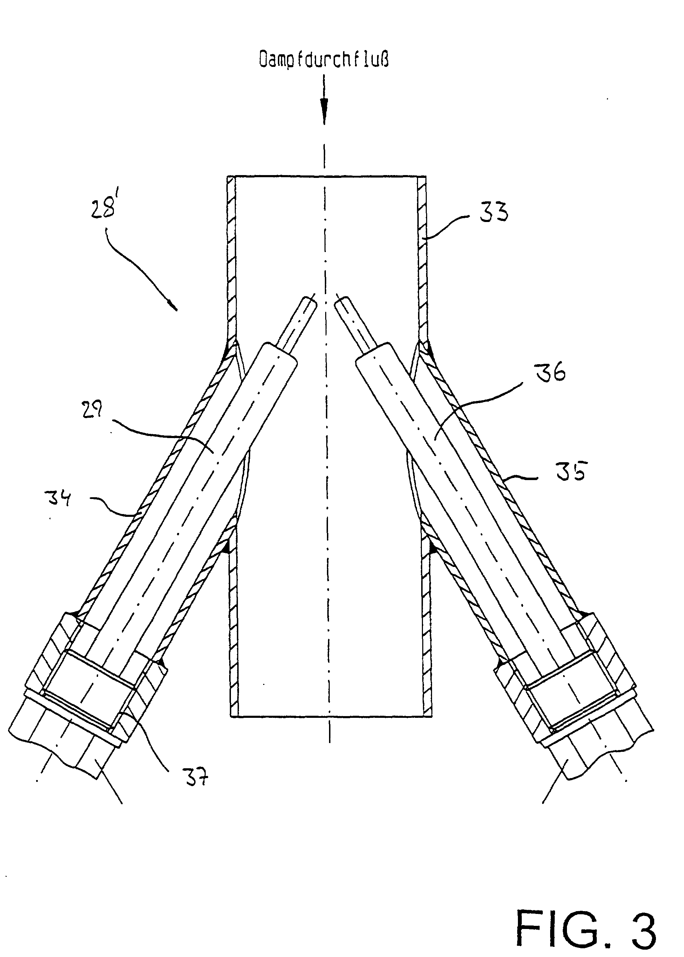

Der zur Aufnahme des Referenzmeßwertaufnehmers 29 vorgesehene Anschlußstutzen 28 kann beispielsweise, wie in Fig.3 dargestellt, aufgebaut sein. Der Anschlußstutzen 28 umfaßt einen Hauptleitungsabschnitt 33, der in den in Fig.1 gezeigten Ablaßrohrleitungsabschnitt 17 integriert ist. Von diesem Hauptleitungsabschnitt 23 zweigen symmetrisch zu einer Mittelachse des Hauptleitungsabschnittes 33 zwei Rohrstutzen 34, 35 ab. In den Rohrstutzen 35 ist der für den Regelbetrieb des Sterilisators vorgesehene Haupt-Temperatursensor 36 angeordnet. In dem Rohrstutzen 34 ist der nur zur Durchführung des Sterilisator-Prüfzyklus vorgesehene Referenzmeßwertaufnehmer 29 eingesetzt. Der Haupttemperatursensor 36 und der Meßwertaufnehmer 29 sind derart angeordnet, daß keine einbaubedingten Unterschiede bei den erfaßten Meßwerten auftreten können. Zur Aufnahme des Referenzmeßwertaufnehmers 29 in dem Rohrstutzen 34 ist ein Gewindeabschnitt 37 vorgesehen, in welchen der Referenzmeßwertaufnehmer 29 abdichtend eingeschraubt ist. Die Rohrstutzen 34 und 35 sind einschließlich der darin vorgesehenen Gewindeabschnitte im wesentlichen baugleich ausgebildet. Bei der hier dargestellten Ausführungsform arbeiten der Haupttemperatursensor 36 sowie der Referenzmeßwertaufnehmer 29 nach dem gleichen Meßprinzip. Die durch die beiden Meßwertaufnehmer erfaßten Meßwerte sind bei dieser Anordnung unmittelbar miteinander vergleichbar.

Es ist jedoch auf möglich, bauartverschiedene Meßwertaufnehmer in die entsprechenden Rohrstutzen 34, 35 einzuschrauben und erst die entsprechend verarbeiteten, unterschiedlichen Meßsignale der beiden Meßwertaufnehmer zu vergleichen. Der Referenzmeßwertaufnehmer kann hierzu beispielsweise unter Zwischenschaltung einer analog und/oder digital arbeitenden Schaltung an die Steuereinheit angeschlossen werden. Insbesondere die zur Erfassung der Temperatur vorgesehen Meßwertaufnehmer und der Referenzmeßwertaufnehmer können auch in der Sterilisatorkammer angeordnet sein.

Der erfindungsgemäße Sterilisator ist gemäß einer bevorzugten Ausführungsform mit einer Anzeigeeinrichtung, vorzugsweise einem LCD-Display, versehen, in welchem die jeweils von einem Systemtechniker an dem Sterilisator vorzunehmenden Konfigurationsänderungen angegeben werden.

Ein in Verbindung mit einer derartigen Benutzerführung durchgeführter Überwachungsvorgang gestaltet sich beispielsweise wie folgt:

Zunächst wird seitens eines Servicetechnikers eine an einem Sterilisator vorgesehene Inspektionsklappe geöffnet. Das Öffnen der Inspektionsklappe wird über einen an die Steuereinheit SPS angeschlossenen Schalter erfaßt. Bei geöffneter Inspektionsklappe fordert die Steuereinheit SPS unmittelbar zur Eingabe eines Berechtigungsnachweises auf. Dieser Berechtigungsnachweis kann seitens des Servicetechnikers beispielsweise durch Eingabe eines Zahlencodes oder durch Einführen einer Magnetkarte in eine ebenfalls mit der Steuereinheit SPS verbundene Leseeinrichtung erfolgen. Nach Eingabe der Zugangsberechtigung kann angegeben werden, ob der Sterilisator im Normalbetrieb betrieben werden soll, oder ob die Durchführung eines Kontrollzyklus gewünscht wird. Wird seitens des Servicetechnikers die Durchführung eines Kontrollzyklus ausgewählt, so wird seitens der Steuereinheit SPS weiter abgefragt, ob ausschließlich die Drucksensoren, ausschließlich die Temperatursensoren oder alle Sensoren (Komplettabgleich) geprüft werden sollen. Wird beispielsweise ein Komplettabgleich ausgewählt, so fordert die Steuereinheit SPS dazu auf, die Referenzmeßwertaufnehmer 25 und 29 in die entsprechenden Meßstellen des Sterilisators einzusetzen und die Anschlußleitungen der Referenzmeßwertaufnehmer an die Steuereinheit SPS anzuschließen. Werden diese Aufforderungen durch den Servicetechniker quittiert, wird seitens der Steuereinheit überprüft, ob die Referenzsensoren an die entsprechenden Schnittstellen angeschlossen sind.

Im Falle eines positiven Prüfergebnisses wird seitens der Steuereinheit SPS ein Evakuier-Vorgang auf einen Kammerdruck von 60 mbar veranlaßt. Anschließend wird ein Temperaturabgleich durchgeführt. Dies erfolgt, indem der Sterilisatorkammer 2 kontinuierlich Dampf zugeführt wird. Während dieser Dampfzufuhr bleibt der Regelungszyklus zur Überwachung des Druckes im Inneren des Mantelraumes 3 des Sterilisators aktiv. Die Steuerung der Zufuhr des Dampfes zu der Sterilisatorkammer erfolgt ähnlich wie im Rahmen eines Standardbehandlungsvorganges mit dem Unterschied, daß bestimmte, fest vorgegebene Solldrücke exakt angesteuert werden. Die Druckstufung kann in Schritten von beispielsweise 500 mbar erfolgen. Bei Erreichen der Drücke von 500 mbar, 1000 mbar, 1500 mbar, 2000 mbar, 2500 mbar und 3000 mbar können beispielsweise die Temperaturen 81°C, 100°C, 111°C, 120°C, 127°C und 133°C abgeglichen werden. Der Abgleich dieser Temperaturwerte erfolgt nach Ablauf einer vorbestimmten Haltezeit, innerhalb welcher der Druck im Inneren der Sterilisatorkammer weitgehend konstant gehalten wird. Werden innerhalb dieser Haltezeit Temperaturwerte ermittelt, die außerhalb eines vorbestimmten Toleranzbereiches liegen, werden diese zunächst verworfen und die Haltezeit wird um beispielsweise 5 Sekunden verlängert. Wird innerhalb dieser verlängerten Haltezeit wiederum kein geeigneter Temperaturwert ermittelt, so kann die Haltezeit mehrfach verlängert werden. Wird auch nach einer vorgegebenen maximalen Verlängerung der Haltezeit um beispielsweise 25 Sekunden noch kein zulässiger Temperaturwert ermittelt, so wird das Kontrollprogramm mit dem Hinweis "Korrekturwerte Temperatur zu groß" bzw. "Korrekturwerte Temperatur nicht konstant" abgebrochen. Anderenfalls werden auf Grundlage der durch die entsprechenden Referenzmeßwertaufnehmer ermittelten Temperaturwerte Korrekturwerte errechnet, anhand welcher im späteren Regelbetrieb die durch den fest eingebauten Temperaturmeßwertaufnehmer 18 ermittelten Temperaturmeßwerte korrigiert werden.

Im Anschluß an einen derart durchgeführten Temperaturabgleich erfolgt der Verfahrensschritt des Durchdämpfens bei einem Druck von ca. 3.000 mbar. Dieser Verfahrensschritt ist wichtig, wenn nur ein einziger Druckabgleich durchgeführt werden soll (Dampfzufuhr in die Kammer nach dem Evakuieren). Dieser Verfahrensschritt weicht vom Durchdämpfen im Standardprogramm insoweit ab, als daß der von den Druckaufnehmern 12 und 13 in der Kammer erfaßte Druck ohne Korrektur verwendet wird, und der Verfahrensschritt des Durchdämpfens beendet wird, wenn in der Kammer ein Druck von 3000 mbar erreicht ist.

Anschließend erfolgt ein Prüfzyklus zur Durchführung eines Überdruck-Abgleiches. Hierzu werden zunächst der durch den Referenzmeßwertaufnehmer 25 ermittelte Referenzdruck und der durch die beiden Drucksensoren 12 und 13 erfaßte Druck aufgezeichnet. Anschließend erfolgt eine Druckentlastung in Schritten von beispielsweise jeweils 500 mbar unter Ermittlung der seitens der Drucksensoren 12 und 13 zum jeweiligen Referenzdruckpegel ermittelten Druckwerte sowie ein Abgleich der entsprechenden Meßwertaufnehmer.

Sobald der Druck im Inneren der Sterilisatorkammer auf Umgebungsdruck abgesenkt ist, wird die Sterilisatorkammer belüftet.

Anschließend wird der Druck im Inneren der Kammer wiederum in vorbestimmten Stufen auf einen Druck von beispielsweise 60 mbar abgesenkt. Zu den jeweiligen, durch den Referenzmeßwertaufnehmer 25 aufgenommenen Druckpegeln werden wiederum die entsprechenden Druckpegel der Drucksensoren 12 und 13 ermittelt. Anschließend wird die Sterilisatorkammer erneut belüftet. Sobald im Inneren der Sterilisatorkammer 2 weitgehend Umgebungsdruck hergestellt ist, erfolgt seitens der Steuereinheit SPS die Aufforderung zum Abklemmen der Referenzsensoren, zum Schließen der entsprechenden Anschlußstutzen und zum Schließen der Inspektionsöffnung.

Nach Schließen der Inspektionsöffnung kann über eine an die Steuereinheit angeschlossene Druckereinrichtung ein Meßprotokoll erzeugt werden, in welchem die zu den jeweiligen Referenzmeßwerten durch die fest eingebauten Betriebs-Meßwertaufnehmer 18, 12 und 13 ermittelten Meßwerte graphisch dargestellt sind. In diesem Meßprotokoll sind vorzugsweise auch der zu jedem Referenzmeßwert zugehörige Toleranzbereich sowie die seitens der Steuereinheit SPS ermittelten Korrekturfunktionen graphisch dargestellt.

Die Inspektionsklappe des Sterilisators kann über ein mechanisches Schloß und/oder über eine an die Steuereinheit SPS angeschlossene, elektrisch gesteuerte Sperreinrichtung verriegelt werden. Die Steuereinheit kann derart ausgebildet sein, daß diese eine Rückkehr in den normalen Steuerungsbetrieb des Sterilisators nur dann zuläßt, wenn nach einem Schließen der Inspektionsöffnung ein entsprechender Freigabecode seitens eines Servicetechnikers eingegeben wird.

## Patentansprüche

1. Sterilisator mit einer Sterilisatorkammer, einer Leitungseinrichtung zur Zuund/oder Ableitung eines Prozeßmediums zu oder aus der Sterilisatorkammer, einem Meßwertaufnehmer zur Ermittlung wenigstens einer Zustandsgröße des Prozeßmediums, einer Steuereinrichtung zur Verarbeitung eines von dem Meßwertaufnehmer aufgenommenen Meßwertes und zur Steuerung der Zu- und/oder Ableitung des Prozessmediums aus der Sterilisatorkammer, **dadurch gekennzeichnet, daß** eine Referenzmeßstelle vorgesehen ist, zur Aufnahme eines Referenzmeßwertaufnehmers zur Ermittlung eines Referenzmeßwertes, wobei der Referenzmeßwertaufnehmer mit der Steuereinrichtung verbindbar ist und der Sterilisator in einem Referenz-Betriebsmodus betreibbar ist in welchem die von dem ersten Meßwertaufnehmer aufgenommenen Meßwerte auf Grundlage der durch den Referenzmeßwertaufnehmer aufgenommenen Meßwerte abgleichbar sind.

2. Sterilisator nach Anspruch 1, **dadurch gekennzeichnet, daß** der erste Meßwertaufnehmer und der Referenzmeßwertaufnehmer jeweils Drucksensoren sind.

3. Sterilisator nach Anspruch 1, **dadurch gekennzeichnet, daß** der erste Meßwertaufnehmer und der Referenzmeßwertaufnehmer jeweils Temperatursensoren sind.

4. Sterilisator nach Anspruch 1, **dadurch gekennzeichnet, daß** der erste Meßwertaufnehmer und der Referenzmeßwertaufnehmer jeweils Volumenstromerfassungseinrichtungen, Meßwertaufnehmer für Feuchte, Konzentrationen oder Inertgasanteile sind.

5. Sterilisator nach einem der Ansprüche 1 bis 4, dadurch gekenzeichnet, daß der erste Meßwertaufnehmer und der Referenzmeßwertaufnehmer nach dem gleichen Meßprinzip arbeiten.

6. Sterilisator nach einem der Ansprüche 1 bis 4, **dadurch gekennzeichnet, daß** der Referenzmeßwertaufnehmer derart ausgebildet ist, daß dieser innerhalb des Meßbereichs des ersten Meßwertaufnehmers wenigstens zwei Referenzmeßwerte liefert.

7. Sterilisator nach einem der Ansprüche 1 bis 6, **dadurch gekennzeichnet, daß** die Steuereinrichtung eine Datenverarbeitungseinrichtung umfaßt, die die Meßwertauswertung der Meßwerte des ersten Meßwertaufnehmers auf Grundlage der durch den Referenzmeßwertaufnehmer aufgenommenen Meßwerte aktualisiert oder korrigiert .

8. Sterilisator nach einem der Ansprüche 1 bis 7, **dadurch gekennzeichnet, daß** eine Prüfeinrichtung vorgesehen ist die eine Abweichung zwischen den durch den ersten Meßwertaufnehmer aufgenommenen Meßwerten und den durch den Referenzmeßwertaufnehmer aufgenommenen Referenzmeßwerten mit vorgegebenen Referenz-Abweichungen vergleicht.

9. Sterilisator nach wenigstens einem der Ansprüche 1 bis 8, **dadurch gekennzeichnet, daß** die Prüfeinrichtung in die Datenverarbeitungseinrichtung integriert ist.

10. Sterilisator nach wenigstens einem der Ansprüche 1 bis 9, **dadurch gekennzeichnet, daß** die Referenz-Abweichungen in der Datenverarbeitungseinrichtung gespeichert sind.

11. Verfahren zur Überwachung eines Meßwertaufnehmers eines Sterilisators zur Sterilisationsbehandlung von Sterilgut in einem Sterilisator unter Verwendung eines gasoder dampfförmigen Prozeßmediums bei welchem
- an einer ersten Meßstelle die Temperatur oder der Druck des Prozeßmediums mittels einer Sensoreinrichtung erfaßt und ein erster Temperatur- oder Druck-Meßwert ermittelt wird,
- an einer zweiten Meßstelle mittels einer zweiten Sensoreinrichtung ein zweiter Temperatur- oder Druck-Meßwert ermittelt wird,
- der ermittelte erste Meßwert und der ermittelte zweite Meßwert einer Datenverarbeitungseinrichtung zugeführt werden, und
- der ermittelte zweite Meßwert mit dem ermittelten ersten Meßwert verglichen wird.

12. Verfahren nach Anspruch 11, **dadurch gekennzeichnet, daß** als zweite Sensoreinrichtung eine Referenzsensoreinrichtung verwendet wird.

13. Verfahren nach Anspruch 11 oder 12, **dadurch gekennzeichnet, daß** die zweite Sensoreinrichtung zur Durchführung eines Kontrollvorganges in die zweite Meßstelle eingebracht wird und nach Durchführung des Kontrollvorganges von der zweiten Meßstelle entfernt wird.

14. Verfahren nach einem der Ansprüche 11 bis 13, **dadurch gekennzeichnet, daß** die zweite Sensoreinrichtung zur Durchführung des Kontrollvorganges mit einer Steuereinrichtung des Sterilisators verbunden und nach Durchführung des Kontrollvorganges von der Steuereinrichtung getrennt wird.

15. Verfahren nach wenigstens einem der Ansprüche 11 bis 13, **dadurch gekennzeichnet, daß** während eines Kontrollvorganges mehrere Referenzmeßwerte ermittelt werden, und daß auf Grundlage der ermittelten Meßwerte die Meßwertauswertung bezüglich des ersten Meßwertaufnehmers aktualisiert wird.

16. Verfahren nach wenigstens einem der Ansprüche 11 bis 15, **dadurch gekennzeichnet, daß** ein Kontrollzyklus zur Ermittlung der Referenzmeßwerte mehrfach durchgeführt wird.

17. Verfahren nach wenigstens einem der Ansprüche 11 bis 16, **dadurch gekennzeichnet, daß** der Kontrollzyklus durch die Steuereinheit gesteuert wird.

18. Verfahren nach wenigstens einem der Ansprüche 11 bis 17, **dadurch gekennzeichnet, daß** zur Durchführung des Kontrollzyklus eine Berechtigungsabfrage durchgeführt wird.

19. Verfahren nach Anspruch 18, **dadurch gekennzeichnet, daß** die ermittelten Referenzmeßwerte in der Steuereinrichtung aufgezeichnet werden.

20. Verfahren nach einem der Ansprüche 11 bis 19, **dadurch gekennzeichnet, daß** die Referenzwerte unter Veränderung weiterer Parameter ermittelt werden.

21. Verfahren nach einem der Ansprüche 11 bis 20, **dadurch gekennzeichnet, daß** ein Meßwert-Abgleich des ersten Meßwertaufnehmers mit dem Refereozmeßwertaufnehmer durch Errechnung einer Korrekturfunktion erfolgt.

22. Verfahren nach einem der Ansprüche 11 bis 20, **dadurch gekennzeichnet, daß** ein Meßwert-Abgleich des ersten Meßwertaufnehmers mit dem Referenzmeßwertaufnehmer durch Änderung des Meßwertaufnahmeverhaltens des ersten Meßwertaufnehmers erreicht wird.

## Claims

1. Steriliser with a steriliser chamber, conveying means for conveying a process medium into and/or out of the steriliser chamber, a measurement recorder for determining at least one state variable of the process medium, control means for processing a measurement recorded by the measurement recorder and for controlling the conveying of the process medium into and/or out of the steriliser chamber, **characterised in that** a reference measuring point is provided, for accommodating a reference measurement recorder for determining a reference measurement, the reference measurement recorder being connectable to the control means and the steriliser being capable of operating in a reference operating mode in which the measurements recorded by the first measurement recorder can be compared on the basis of the measurements recorded by the reference measurement recorder.

2. Steriliser according to claim 1, **characterised in that** the first measurement recorder and the reference measurement recorder are both pressure sensors.

3. Steriliser according to claim 1, **characterised in that** the first measurement recorder and the reference measurement recorder are both temperature sensors.

4. Steriliser according to claim 1, **characterised in that** the first measurement recorder and the reference measurement recorder are both volume flow detectors, measurement recorders for moisture, concentrations or inert gas contents.

5. Steriliser according to one of claims 1 to 4, **characterised in that** the first measurement recorder and the reference measurement recorder operate on the same measuring principle.

6. Steriliser according to one of claims 1 to 4, **characterised in that** the reference measurement recorder is constructed so as to yield at least two reference measurements within the measuring range of the first measurement recorder.

7. Steriliser according to one of claims 1 to 6, **characterised in that** the control apparatus comprises a data processing apparatus which updates or corrects the evaluation of the measurements from the first measurement recorder on the basis of the measurements recorded by the reference measurement recorder.

8. Steriliser according to one of claims 1 to 7, **characterised in that** a test device is provided which compares any deviation between the measurements recorded by the first measurement recorder and the reference measurements recorded by the reference measurement recorder with predetermined reference deviations.

9. Steriliser according to at least one of claims 1 to 8, **characterised in that** the test device is integrated in the data processing apparatus.

10. Steriliser according to at least one of claims 1 to 9, **characterised in that** the reference deviations are stored in the data processing apparatus.

11. Method of monitoring a measurement recorder of a steriliser for sterilisation treatment of sterile material in a steriliser using a process medium in gaseous or vapour form, wherein
- at a first measuring point the temperature or the pressure of the process medium is detected by a sensor device and a first temperature or pressure measurement is obtained,
- at a second measuring point using a second sensor device a second temperature or pressure measurement is obtained,
- the first measurement obtained and the second measurement obtained are fed into a data processing apparatus, and
- the second measurement obtained is compared with the first measurement obtained.

12. Method according to claim 11, **characterised in that** a reference sensor device is used as the second sensor device.

13. Method according to claim 11 or 12, **characterised in that** the second sensor device is placed in the second measuring point in order to carry out a monitoring operation and after the monitoring operation has been carried out it is removed from the second measuring point.

14. Method according to one of claims 11 to 13, **characterised in that** the second sensor device is connected to a control apparatus of the steriliser in order to carry out the monitoring operation and after the monitoring operation has been carried out it is separated from the control apparatus.

15. Method according to at least one of claims 11 to 13, **characterised in that** during a monitoring operation a plurality of reference measurements are obtained and **in that** the measurement evaluation with regard to the first measurement recorder is updated on the basis of the measurements obtained.

16. Method according to at least one of claims 11 to 15, **characterised in that** a control cycle is run several times in order to obtain the reference measurements.

17. Method according to at least one of claims 11 to 16, **characterised in that** the control cycle is controlled by the control unit.

18. Method according to at least one of claims 11 to 17, **characterised in that** in order to run the control cycle an authorisation interrogation is carried out.

19. Method according to claim 18, **characterised in that** the reference measurements obtained are recorded in the control apparatus.

20. Method according to one of claims 11 to 19, **characterised in that** the reference values are determined by changing other parameters.

21. Method according to one of claims 11 to 20, **characterised in that** a comparison of the measurements from the first measurement recorder with the reference measurement recorder is carried out by calculating a correction function.

22. Method according to one of claims 11 to 20, **characterised in that** a comparison of the measurements from the first measurement recorder with the reference measurement recorder is carried out by varying the measurement recording characteristics of the first measurement recorder.

## Revendications

1. Stérilisateur avec une chambre de stérilisateur, un dispositif de tuyauterie pour l'amenée et/ou l'évacuation d'un milieu de processus vers ou depuis une chambre de stérilisateur, un capteur de valeur de mesure pour déterminer au moins une grandeur d'état du milieu de processus, un dispositif de commande pour le traitement d'une valeur de mesure enregistrée par le capteur de valeur de mesure et pour la commande de l'alimentation et/ou de l'évacuation du milieu de processus hors de la chambre de stérilisateur, **caractérisé en ce qu'**est prévu un point de mesure de référence pour recevoir un capteur de valeur de mesure de référence afin de déterminer une valeur de mesure de référence, le capteur de valeur de mesure de référence étant susceptible d'être relié à un dispositif de commande et le stérilisateur pouvant fonctionner en un mode de fonctionnement de référence dans lequel des valeurs de mesure enregistrées par le premier capteur de valeur de mesure peuvent être équilibrées sur la base des valeurs de mesure enregistrées par le capteur de mesure de référence.

2. Stérilisateur selon la revendication 1, **caractérisé en ce que** le premier capteur de valeur de mesure et le capteur de valeur de mesure de référence sont chacun des capteurs de pression.

3. Stérilisateur selon la revendication 1, **caractérisé en ce que** le premier capteur de valeur de mesure et le capteur de valeur de mesure de référence sont chacun des capteurs de température.

4. Stérilisateur selon la revendication 1, **caractérisé en ce que** le premier capteur de valeur de mesure et le capteur de valeur de mesure de référence sont chacun des dispositifs de détection de débit volumique, des enregistreurs de valeur de mesure pour l'humidité, des concentrations ou des proportions en gaz inerte.

5. Stérilisateur selon l'une des revendications 1 à 4, **caractérisé en ce que** le premier capteur de valeur de mesure et le capteur de mesure de référence fonctionnent selon le même principe de mesure.

6. Stérilisateur selon l'une des revendications 1 à 4, **caractérisé en ce que** le capteur de mesure de référence est réalisé de manière que celui-ci fournisse au moins deux valeurs de mesure de référence dans la plage de mesure du premier capteur de valeur de mesure.

7. Stérilisateur selon l'une des revendications 1 à 6, **caractérisé en ce que** le dispositif de commande comprend un dispositif de traitement de données actualisant ou corrigeant l'évaluation de valeur de mesure obtenue d'après les valeurs de mesure du premier capteur de valeur de mesure, en se basant sur les valeurs de mesure enregistrées par le capteur de valeur de mesure de référence.

8. Stérilisateur selon l'une des revendications 1 à 7, **caractérisé en ce qu'**est prévu un dispositif d'essai comparant un écart entre les valeurs de mesure enregistrées par le premier capteur de valeur de mesure et les valeurs de mesure de référence enregistrées par le capteur de valeur de mesure de référence, à des écarts de référence prédéterminés.

9. Stérilisateur selon au moins l'une des revendications 1 à 8, **caractérisé en ce que** le dispositif d'essai est intégré dans le dispositif de traitement des données.

10. Stérilisateur selon au moins l'une des revendications 1 à 9, **caractérisé en ce que** les écarts de référence sont mémorisés dans le dispositif de traitement de données.

11. Procédé de surveillance d'un capteur de valeur de mesure d'un stérilisateur pour le traitement par stérilisation de produits stérilisés, dans un stérilisateur, avec utilisation d'un milieu de processus gazeux ou sous forme de vapeur, pour lequel
- en un premier point de mesure on mesure la température ou la pression du fluide de processus à l'aide d'un dispositif capteur et on détermine une première valeur de mesure de température ou de pression,
- en une deuxième point de mesure, au moyen d'un deuxième dispositif capteur, on détermine une deuxième valeur de mesure de température ou de pression,
- la première valeur de mesure déterminée et la deuxième valeur de mesure déterminée sont amenées à un dispositif de traitement des données, et
- la deuxième valeur de mesure déterminée est comparée à la première valeur de mesure déterminée.

12. Procédé selon la revendication 11, **caractérisé en ce qu'**on utilise comme deuxième dispositif capteur un dispositif capteur de référence.

13. Procédé selon la revendication 11 ou 12, **caractérisé en ce que** le deuxième dispositif capteur devant effectuer un processus de contrôle est inséré dans le deuxième point de mesure et est enlevé du deuxième point de mesure une fois que le processus de contrôle a été effectué.

14. Procédé selon l'une des revendications 11 à 13, **caractérisé en ce que** le deuxième dispositif capteur devant effectuer le processus de contrôle est relié à un dispositif de commande du stérilisateur et est séparé du dispositif de commande une fois que le processus de contrôle a été effectué.

15. Procédé selon au moins l'une des revendications 11 à 13, **caractérisé en ce que** pendant un processus de contrôle, plusieurs valeurs de mesure de référence sont déterminées et que l'évaluation de valeur de mesure concernant le premier capteur de valeur de mesure est actualisée d'après les valeurs de mesure déterminées.

16. Procédé selon au moins l'une des revendications 11 à 15, **caractérisé en ce qu'**un cycle de contrôle est effectué à plusieurs reprises pour déterminer les valeurs de mesure de référence.

17. Procédé selon au moins l'une des revendications 11 à 16, **caractérisé en ce que** le cycle de contrôle est commandé par l'unité de commande.

18. Procédé selon au moins l'une des revendications 11 à 17, **caractérisé en ce qu'**une interrogation d'autorisation est effectuée pour procéder au cycle de contrôle.

19. Procédé selon la revendication 18, **caractérisé en ce que** les valeurs de mesure de référence déterminées sont enregistrées dans le dispositif de commande.

20. Procédé selon au moins l'une des revendications 11 à 19, **caractérisé en ce que** les valeurs de référence sont déterminées en modifiant d'autres paramètres.

21. Procédé selon au moins l'une des revendications 11 à 20, **caractérisé en ce qu'**un équilibrage de valeur de mesure du premier capteur de valeur de mesure est effectué avec le capteur de valeur de mesure de référence par calcul d'une fonction de correction.

22. Procédé selon au moins l'une des revendications 11 à 20, **caractérisé en ce qu'**un équilibrage de valeur de mesure du premier capteur de valeur de mesure avec le capteur de valeur de mesure de référence est obtenu par modification du comportement en enregistrement de valeur de mesure du premier capteur de valeur de mesure.
